(19) Europäisches Patentamt
European Patent Office
Office européen des brevets

(11) EP 2 359 860 B1

(12) **EUROPEAN PATENT SPECIFICATION**

(45) Date of publication and mention of the grant of the patent:
**06.08.2014 Bulletin 2014/32**

(21) Application number: **09827766.8**

(22) Date of filing: **23.11.2009**

(51) Int Cl.:
*A61K 47/30* (2006.01) *A61K 31/335* (2006.01)
*A61P 35/00* (2006.01)

(86) International application number:
**PCT/KR2009/006882**

(87) International publication number:
**WO 2010/059001 (27.05.2010 Gazette 2010/21)**

(54) **POLYMER MICELLE COMPOSITION FOR TREATMENT OF RESISTANT CANCER CELLS**

POLYMERMIZELLENZUSAMMENSETZUNG ZUR BEHANDLUNG RESISTENTER KREBSZELLEN

COMPOSITION DE MICELLES POLYMÈRES POUR LE TRAITEMENT DE CELLULES CANCÉREUSES RÉSISTANTES

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **21.11.2008 KR 20080116141**

(43) Date of publication of application:
**24.08.2011 Bulletin 2011/34**

(73) Proprietor: **Samyang Biopharmaceuticals Corporation**
**Seoul 110-470 (KR)**

(72) Inventors:
- **KANG, Hye-Won**
  **Seoul 143-201 (KR)**
- **SEO, Min Hyo**
  **Daejeon 302-782 (KR)**
- **KIM, Bong Oh**
  **Daejeon 300-070 (KR)**

(74) Representative: **Ferreccio, Rinaldo**
**Botti & Ferrari S.r.l.**
**Via Cappellini, 11**
**20124 Milano (IT)**

(56) References cited:
**WO-A1-01/87345 US-A1- 2004 253 195**

- **ALIABADI HAMIDREZA MONTAZERI ET AL: "Disposition of drugs in block copolymer micelle delivery systems: from discovery to recovery", CLINICAL PHARMACOKINETICS, ADIS INTERNATIONAL LTD., AUCKLAND, NZ, vol. 47, no. 10, 1 October 2008 (2008-10-01), pages 619-634, XP009151392, ISSN: 0312-5963**
- **MALINGRE, M.M. ET AL.: 'Coadministration of Cyclosporine Strongly Enhances the Oral Bioavailability of Docetaxel' JOURNAL OF CLINICAL ONCOLOGY 2001, pages 1160 - 1166, XP008149347**
- **ALIABADI, H.M. ET AL.: 'Micelles of methoxy poly (ethylene oxide)-b-poly (epsilone- caprolactone) as vehicles for the solubilization and controlled delivery of cyclosporine A' J JOURNAL OF CONTROLLED RELEASE vol. 104, 2005, pages 301 - 311, XP027664273**
- **SHUAI , X. ET AL.: 'Core-Cross-Linked Polymeric Micelles as Paclitaxel Carriers' BIOCONJUGATE CHEMISTRY vol. 15, 2004, pages 441 - 448, XP001195317**
- **LETCHFORD, K. ET AL.: 'A review of the formulation and classification of amphiphilic block copolymer nanoparticulate structures: micelles, nanospheres, nanocapsules and polymersomes' EUROPEAN JOURNAL OF PHARMACEUTICS AND BIOPHARMACEUTICS 2007, pages 259 - 269, XP005914364**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### TECHNICAL FIELD

The present disclosure relates to a polymer micelle composition for treatment of resistant cancer and a method for preparing the same.

### BACKGROUND ART

Taxane anti-cancer agents are successfully used in the treatment of breast cancer, ovarian cancer, lung cancer, prostate cancer, or the like. However, since the taxane anti-cancer agents paclitaxel and docetaxel are very poorly soluble in water, they need to be solubilized, for example, by a nonionic surfactant. The agents are commercially available as Taxol® or Taxotere®, which are solubilized by a nonionic surfactant such as Cremophor and polysorbate. Although Taxol® and Taxotere® show superior anti-cancer effects, they have side effects such as hypersensitive reactions caused by the use of the solubilizer. Thus, researches are ongoing about new agents with no side effect.

Although many anti-cancer agents including taxanes are widely used because of their superior anti-cancer effect, resistance to the anti-cancer agents causes problems. There are various mechanisms by which cancer cells exhibit resistance to the anti-cancer agents. One of the well-known mechanisms is the exocytosis of the anti-cancer agent due to overexpression of P-glycoprotein. In order to overcome the cancer resistance caused by P-glycoprotein overexpression, use of an inhibitor of P-glycoprotein is studied.

Verapamil, ketoconazole, cyclosporin, etc. are known as typical P-glycoprotein inhibitors. These substances are studied as P-glycoprotein inhibitor for resolving the reduced oral bioavailability problem of the anti-cancer agent due to the overexpression of P-glycoprotein. Cyclosporin is a poorly soluble drug. A composition using Cremophor as a solubilizer is marketed under the brand name Sandimmune® as a formulation for intravenous injection and oral administration. Also, an orally-administered microemulsion formulation is available under the brand name Neoral®.

The cyclosporin formulation for intravenous injection includes Cremophor as the solubilizer. However, Cremophor is associated with the side effect due to hypersensitive reaction. For this reason, it is very difficult to apply a combination therapy of the taxane anti-cancer agent Taxol® or Taxotere® together with the cyclosporin formulation for intravenous injection Sandimmune® for treating resistant cancer. In addition, when cyclosporin is administered non-specifically, e.g. via intravenous injection, the drug may be delivered to the systemic circulation, resulting in declined immunity of the cancer patient who already has weakened immunity.

Nothing is known about a composition containing both a taxane compound and a P-glycoprotein inhibitor. Moreover, nothing is known about a composition for treating resistant cancer by inhibiting P-glycoprotein.

### DISCLOSURE

### TECHNICAL PROBLEM

The present disclosure is directed to providing a polymer micelle composition for treatment of resistant cancer comprising an amphiphilic diblock copolymer and active ingredients, wherein said active ingredients are taxane and a P-glycoprotein inhibitor, the P-glycoprotein inhibitor being cyclosporine.

The present disclosure is also directed to providing a method for preparing a polymer micelle composition for treatment of resistant cancer comprising an amphiphilic diblock copolymer and active ingredients, wherein said active ingredients are taxane and a P-glycoprotein inhibitor, the P-glycoprotein inhibitor being cyclosporine

### TECHNICAL SOLUTION

In one general aspect, the present disclosure provides a polymer micelle composition for parenteral treatment of resistant cancer comprising an amphiphilic diblock copolymer and active ingredients, wherein said active ingredients are taxane and a P-glycoprotein inhibitor, the P-glycoprotein inhibitor being cyclosporine. In another general aspect, the present disclosure provides a method for preparing the polymer micelle composition for treatment of resistant cancer.

Other features and aspects will be apparent from the following detailed description, the drawings, and the claims.

### ADVANTAGEOUS EFFECTS

The polymer micelle composition disclosure for treatment of resistant cancer according to the present, which comprises the amphiphilic diblock copolymer and, optionally, the polylactic acid alkali metal salt having at least one terminal carboxyl group, can be accumulated in cancer tissue at high concentrations because of the enhanced permeability and retention (EPR) effect. Since it comprises taxane as well as the P-glycoprotein inhibitor, it exhibits superior anti-cancer effects for the cancer cells exhibiting resistance due to overexpression of P-glycoprotein by a taxane anti-cancer agent in the cancer tissue. Furthermore, it does not cause hypersensitive reactions due to the use of a solubilizer. In addition, it can reduce the side effect caused by systemic circulation of cyclosporin by allowing cyclosporin to be predominantly distributed in the cancer tissue.

## DESCRIPTION OF DRAWINGS

The above and other objects, features and advantages of the present disclosure will become apparent from the following description of certain exemplary embodiments given in conjunction with the accompanying drawings, in which:

FIG. 1 shows a result of analyzing the particle size of a polymer micelle in which paclitaxel and cyclosporin are encapsulated together in an aqueous solution by dynamic light scattering as a weight average distribution;
FIG. 2 shows a result of measuring the concentration of paclitaxel and cyclosporin for a polymer micelle in which paclitaxel and cyclosporin are encapsulated together by high-performance liquid chromatography (HPLC);
FIGS. 3 and 4 show the concentration of paclitaxel in blood and brain tissue for a polymer micelle in which paclitaxel and cyclosporin are encapsulated together;
FIG. 5 shows the concentration of paclitaxel in blood for a composition containing a mixture of a paclitaxel-containing polymer micelle and a cyclosporin-containing polymer micelle; and
FIG. 6 shows a result of comparing the anti-cancer effect of polymer micelle compositions.

## MODE FOR INVENTION

The present disclosure a polymer micelle composition for treatment of resistant cancer comprising an amphiphilic diblock copolymer and active ingredients, wherein said active ingredients are taxane and a P-glycoprotein inhibitor, the P-glycoprotein inhibitor being cyclosporine. In an embodiment of the present disclosure, the polymer micelle composition for treatment of resistant cancer may comprise taxane, cyclosporin and an amphiphilic diblock copolymer to solubilize the taxane and cyclosporin.

In another embodiment, the polymer micelle may be a nanoparticle. Specifically, the micelle or nanoparticle may have a particle diameter of 10-200 nm.

Since taxane and cyclosporin are both poorly soluble in water, special technique and composition for solubilization are required for administration to the human body. Usually, the organic solvent ethanol and the surfactant Cremophor or polysorbate are used to solubilize the drugs. However, since the two surfactants may cause severe hypersensitive reaction when administered, for example, intravenously, caution and pretreatment are required. The present disclosure provides a composition with no side effect such as hypersensitive reaction even when taxane and cyclosporin are administered together intravenously.

The term "resistant cancer" as used in the present disclosure collectively refers to a cancer that has developed resistance to an anti-cancer agent due to its use. More specifically, it refers to a cancer that has developed resistance due to overexpression of P-glycoprotein. The cancer includes all type of cancers, e.g. breast cancer, ovarian cancer, lung cancer, prostate cancer, etc., without any special restriction.

The term "nanoparticle" as used in the present disclosure refers to a particle having a nano-scale particle diameter. It may include a micelle-structured particle.

The term "mixed" used to describe "mixed micelle", "mixed nanoparticle", "mixed micelle or nanoparticle", or the kind of micelle in the present disclosure refers to a state wherein one drug is encapsulated in one micelle or nanoparticle, and a taxane-containing micelle and a cyclosporin-containing micelle are mixed. The mixed micelle or nanoparticle may be obtained by preparing a taxane-containing micelle and a cyclosporin-containing micelle or nanoparticle separately and then mixing them.

The expression "complex" used to describe "complex micelle", "complex nanoparticle", "complex micelle or nanoparticle", or the kind of micelle in the present disclosure refers to a state wherein taxane and cyclosporin are encapsulated together in one micelle or nanoparticle. The complex micelle or nanoparticle may be obtained encapsulating the two drugs in a micelle or nanoparticle core at the same time.

The polymer micelle composition according to the present disclosure includes both the mixed and complex polymer micelles encapsulating taxane and cyclosporin. In an embodiment, the anti-cancer agent composition for treatment of resistant cancer may comprise the active ingredients taxane and cyclosporin encapsulated in a micelle formed from an amphiphilic diblock copolymer. Specifically, the composition may be either a complex micelle form wherein taxane and cyclosporin are encapsulated together in one micelle formed from the amphiphilic diblock copolymer, or a mixed form wherein taxane and cyclosporin are encapsulated in different micelles. In another embodiment, the composition may be a mixture of: a composition comprising taxane and cyclosporin together in one micelle; and a composition comprising a mixture of a taxane-containing polymer micelle and a cyclosporin-containing polymer micelle.

In an embodiment, the taxane may be paclitaxel, docetaxel, 7-epipaclitaxel, t-acetyl paclitaxel, 10-desacetyl paclitaxel, 10-desacetyl-7-epipaclitaxel, 7-xylosyl paclitaxel, 10-desacetyl-7-glutaryl paclitaxel, 7-N,N-dimethylglycyl paclitaxel, 7-L-alanyl paclitaxel or a mixture thereof. Specifically, it may be paclitaxel or docetaxel. The taxane may be either amorphous or crystalline. Further, it may be either an anhydride or a hydrate. In another embodiment, the taxane may be an anhydride of paclitaxel or docetaxel.

The cyclosporin serves to suppress overexpression of P-glycoprotein. Cyclosporin A, B, C or D may be used. In an embodiment, the cyclosporin may be cyclosporin A. Cyclosporin A is a cyclic peptide consisting of 11 amino acids, and has various physiological activities including anti-bacterial, anti-parasitic, and immune-suppressing activities. Especially, cyclosporin A is widely used for tissue or organ transplantation and treatment of autoimmune diseases because of its immunosuppressing ability.

In an anti-cancer agent composition according to an embodiment of the present disclosure, the weight ratio of taxane to cyclosporin (weight of taxane / weight of cyclosporin) may be from 0.1 to 2.0, more specifically from 0.8 to 1.5. Within this weight ratio range, the effect of taxane as the anti-cancer agent and the effect of cyclosporin as the P-glycoprotein inhibitor may be optimized.

In another embodiment, the combined content of taxane and cyclosporin may be 0.1-20 wt%, specifically 0.2-10 wt%, based on the total weight of the composition. Since taxane and cyclosporin are encapsulated in the polymer micelle, the content of taxane and cyclosporin that can be encapsulated in the polymer micelle is limited. In an embodiment of the present disclosure, taxane may be included in an amount of 0.01-10 wt%, specifically 0.01-5 wt%, and cyclosporin may be included in an amount of 0.01-10 wt%, specifically 0.01-5 wt%, based on the total weight of the composition.

In an embodiment, the amphiphilic diblock copolymer may be an A-B type diblock copolymer comprising a hydrophilic block A and a hydrophobic block B. The hydrophilic block A may be polyethylene glycol, and the hydrophobic block B may be polylactic acid or a derivative thereof.

The polyethylene glycol as the hydrophilic block A may be one or more selected from a group consisting of polyethylene glycol and methoxypolyethylene glycol, but without being limited thereto. Specifically, it may be methoxypolyethylene glycol.

The polylactic acid or its derivative as the hydrophobic block B may be one or more selected from a group consisting of polylactic acid, polylactide, polyglycolide, polymandelic acid, polycaprolactone, polydioxan-2-one, polyamino acid, polyorthoester, polyanhydride and a copolymer thereof. Specifically, it may be polylactic acid, polylactide, polyglycolide, polymandelic acid, polycaprolactone or polydioxan-2-one. More specifically, the polylactic acid or its derivative may be one or more selected from a group consisting of polylactic acid, polylactide, polycaprolactone, a copolymer of lactic acid and mandelic acid, a copolymer of lactic acid and glycolic acid, a copolymer of lactic acid and caprolactone, and a copolymer of lactic acid and 1,4-dioxan-2-one.

In an embodiment, the hydrophilic block A may have a number average molecular weight of 500-20,000 daltons, more specifically 1,000-10,000 daltons. The hydrophobic block B may have a number average molecular weight of 500-10,000 daltons. In another embodiment, the content of the hydrophilic block A may be 40-70 wt%, more specifically 50-65 wt%, based on the total weight of the diblock copolymer. Within this range, the micelle of the amphiphilic diblock copolymer can be maintained stably.

The amount of the amphiphilic diblock copolymer may be 80-99.9 wt%, more specifically 40-90 wt%, based on the total weight of the composition. In an embodiment, the composition may comprise: 0.01-10 wt% of taxane; 0.01-10 wt% of cyclosporin; and 80-99.8 wt% of an amphiphilic diblock copolymer, based on the total weight of the composition. In another embodiment, the composition may comprise: 0.01-10 wt% of taxane; 0.01-10 wt% of cyclosporin; 40-90 wt% of an amphiphilic diblock copolymer; and 10-50 wt% of a polylactic acid alkali metal salt having a terminal carboxyl group.

The complex amphiphilic diblock copolymer micelle composition in which taxane and cyclosporin are encapsulated together may have a particle size of 10-200 nm in an aqueous solution, and may be in solid state when freeze dried.

In another embodiment of the present disclosure, the polymer micelle composition for treatment of resistant cancer may further comprise a polylactic acid alkali metal salt having at least one terminal carboxyl group, in addition to the taxane, the cyclosporin and the amphiphilic diblock copolymer. The polylactic acid alkali metal salt keeps the inside of the micelle core in which the drug is incorporated strongly and thus improves the encapsulation efficiency. Accordingly, the present disclosure provides a polymer micelle composition for treatment of resistant cancer comprising taxane, cyclosporin, an amphiphilic diblock copolymer, and a polylactic acid alkali metal salt having at least one terminal carboxyl group. The polylactic acid alkali metal salt refers to a salt formed by an ionic bonding between the terminal carboxyl group and an alkali metal ion.

Specifically, the polylactic acid alkali metal salt having at least one terminal carboxyl group may be represented by Chemical Formula 1:

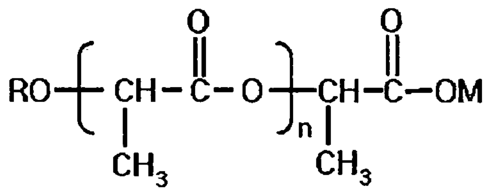

(1)

wherein

R is hydrogen, acetyl, benzoyl, decanoyl, palmitoyl, methyl or ethyl,
M is sodium, potassium or lithium, and
n is an integer from 5 to 35, specifically from 10 to 30.

Specifically, the polylactic acid alkali metal salt may have one terminal carboxyl group.

The other terminus of the polylactic acid alkali metal salt may be substituted with a substituent selected from a group consisting of hydroxyl, acetoxy, benzoyloxy, decanoyloxy, palmitoyloxy and alkoxy. The polylactic acid alkali metal salt is dissolved in an aqueous solution and forms a micelle as the hydrophilic (carboxylate) moiety and the hydrophobic (polylactic acid) moiety of the polylactic acid alkali metal salt keep balance. Thus, when the molecular weight of the hydrophobic moiety is too large, the micelle may not be formed easily since the carboxylates of the hydrophilic moieties aggregate together. And, when the molecular weight is too small, the polylactic acid alkali metal salt may be completely dissolved in water and thus the micelle may not be formed. In an embodiment, the polylactic acid alkali metal salt may have a number average molecular weight of 500-2,500 daltons, specifically 1,000-2,000 daltons. When the molecular weight is smaller than 500 daltons, the polylactic acid alkali metal salt may be completely dissolved in water and thus the micelle may not be formed. And, when the molecular weight exceeds 2,500 daltons, the polylactic acid alkali metal salt may not be dissolved well in an aqueous solution, making the formation of the micelle difficult.

In an embodiment of the present disclosure, the alkali metal of the polylactic acid alkali metal salt may be a monovalent metal such as sodium, potassium or lithium.

The polymer micelle composition may comprise: 0.1-20 wt%, specifically 0.2-10 wt%, of taxane and cyclosporin, respectively; 40-90 wt%, specifically 45-74 wt%, of an amphiphilic diblock copolymer; and 10-50 wt%, specifically 25-45 wt%, of a polylactic acid alkali metal salt having at least one terminal carboxyl group, based on the total weight of the composition.

In an embodiment, a divalent or trivalent metal ion may be added to the micelle composition to further stabilize the polymer micelle formed by mixing the amphiphilic diblock copolymer with the polylactic acid derivative. The divalent or trivalent metal ion is bound to the terminal carboxyl group of the polylactic acid derivative and thus forms a polymer nanoparticle to which the divalent or trivalent metal ion is bound. Accordingly, the present disclosure provides a nanoparticle composition for treatment of resistant cancer comprising taxane, cyclosporin, an amphiphilic diblock copolymer and a polylactic acid having a carboxyl terminus to which a divalent or trivalent metal ion is bound. The divalent or trivalent metal ion substitutes the monovalent metal cation at the carboxyl terminal group of the polylactic acid in the polymer micelle and forms an ionic bonding. The ionic bonding formed by the metal ion further stabilizes the polymer micelle with the strong binding force.

Specifically, the divalent or trivalent metal ion may be selected from calcium, magnesium, barium, chromium, iron, manganese, nickel, copper, zinc, aluminum, etc. More specifically, it may be calcium or magnesium.

The metal ion may be added to the polymer micelle composition in the form of sulfate, hydrochloride, carbonate, phosphate or hydrate. Specifically, calcium chloride, magnesium chloride, zinc chloride, aluminum chloride, iron chloride, calcium carbonate, magnesium carbonate, calcium phosphate, magnesium phosphate, aluminum phosphate, magnesium sulfate, calcium hydroxide, magnesium hydroxide, aluminum hydroxide or zinc hydroxide may be added.

The content of the divalent or trivalent metal ion may be controlled according to the desired release rate of the drug encapsulated in the polymer nanoparticle. Specifically, when the metal ion is included in the polymer nanoparticle composition in an amount less than 1 equivalent based on the carboxyl group of the polylactic acid alkali metal salt, the drug is released quickly. And, when it is included in an amount of 1 equivalent or more based on the carboxyl group of the polylactic acid alkali metal salt, the drug is released slowly.

When the divalent or trivalent metal ion is bound to the terminal carboxyl group of the polylactic acid salt, the composition may comprise: 0.1-20 wt%, specifically 0.2-10 wt%, of taxane and cyclosporin, respectively; 40-90 wt%, specifically 45-74 wt%, of an amphiphilic diblock copolymer; and 10-50 wt%, specifically 25-45 wt%, of a polylactic acid salt having at least one terminal carboxyl group, including the divalent or trivalent metal ion in an amount of 0.01-10 equivalents, specifically 1-2 equivalents, based on the terminal carboxyl group of the polylactic acid salt, based on the total weight of the composition.

The composition for treatment of resistant cancer of the present disclosure may comprise the amphiphilic

diblock copolymer alone, a polymer mixture comprising the amphiphilic diblock copolymer and the polylactic acid alkali metal salt, a polymer mixture comprising the amphiphilic diblock copolymer and the polylactic acid salt having a carboxyl terminus to which the divalent or trivalent metal ion is bound, or a polymer mixture thereof.

The micelle or nanoparticle composition of the present disclosure may be a mixed or complex micelle or nanoparticle composition. Also, the composition of the present disclosure may comprise a micelle comprising an amphiphilic diblock copolymer, a micelle comprising an amphiphilic diblock copolymer and a polylactic acid alkali metal salt, a micelle comprising an amphiphilic diblock copolymer and a polylactic acid salt having a carboxyl terminus to which a divalent or trivalent metal ion is bound, or a mixture thereof.

The present disclosure further provides a method for preparing an anti-cancer agent composition for treatment of resistant cancer. A method for preparing a polymer micelle composition for treatment of resistant cancer comprising taxane and cyclosporin as active ingredients according to an embodiment of the present disclosure may comprise: (a) solubilizing taxane, cyclosporin and an amphiphilic diblock copolymer in an organic solvent; (b) evaporating the organic solvent to prepare a mixture wherein taxane, cyclosporin and the polymer are uniformly mixed; and (c) adding an aqueous solution to the mixture prepared in the step (b) to prepare a polymer micelle wherein taxane and cyclosporin are encapsulated.

A method for preparing a polymer micelle composition for treatment of resistant cancer comprising taxane and cyclosporin as active ingredients according to another embodiment of the present disclosure may comprise: (a) solubilizing taxane, cyclosporin, an amphiphilic diblock copolymer and a polylactic acid alkali metal salt having at least one terminal carboxyl group in an organic solvent; (b) evaporating the organic solvent to prepare a mixture wherein taxane, cyclosporin and the polymer are uniformly mixed; and (c) adding an aqueous solution to the mixture prepared in the step (b) to prepare a polymer micelle wherein taxane and cyclosporin are encapsulated.

Taxane and cyclosporin may be separately solubilized together with the polymer in different organic solvents, and then a resulting taxane-containing polymer micelle composition and a cyclosporin-containing polymer micelle composition thus prepared may be mixed to prepare the mixed micelle composition.

Accordingly, the anti-cancer agent composition according to the present disclosure may be either a complex type wherein taxane and cyclosporin are encapsulated together in the polymer micelle or nanoparticle, or a mixture of a taxane-containing polymer micelle or nanoparticle with a cyclosporin-containing polymer micelle or nanoparticle. More specifically, the two types of compositions may be prepared as follows.

<Preparation of complex nanoparticle composition wherein taxane and cyclosporin are encapsulated together>

This composition is a complex type wherein taxane and cyclosporin are encapsulated together in one micelle or nanoparticle.

A method for preparing a complex anti-cancer agent polymer micelle composition wherein taxane and cyclosporin are encapsulated together as active ingredients may comprise:

(a) solubilizing taxane, cyclosporin, an amphiphilic diblock copolymer and, optionally, a polylactic acid alkali metal salt having at least one terminal carboxyl group in an organic solvent selected from a group consisting of ethanol, methanol, propanol, acetone, acetonitrile, dichloromethane, chloroform, methyl ethyl ketone and ethyl acetate;
(b) evaporating the organic solvent to prepare a mixture wherein taxane, cyclosporin, the polymer and, optionally, the polylactic acid alkali metal salt having at least one terminal carboxyl group are uniformly mixed; and
(c) adding an aqueous solution to the mixture prepared in the step (b) to prepare a polymer micelle composition wherein taxane and cyclosporin are encapsulated in a core.

In an embodiment, when a polylactic acid alkali metal salt having at least one terminal carboxyl group is used to prepare a nanoparticle to which a divalent or trivalent metal ion is bound, the preparation method may further comprise, after the step (c):

(c-1) adding a divalent or trivalent metal ion to the polymer micelle so that it is bound to the terminal group of the polylactic acid salt.

In the step (c-1), an aqueous solution containing 0.001-2 M of a divalent or trivalent metal ion may be added to the aqueous solution of the complex polymer micelle, and the resultant mixture may be slowly stirred at room temperature for 0.1-1 hour to bind the metal ion to the terminal group of the polylactic acid salt via ionic bonding.

In another embodiment, the preparation method may further comprise, after the step (c) or (c-1):

(d) sterilizing the resulting micelle composition;
(e) filling the sterilized micelle composition in a container; and

(f) freeze drying the micelle composition filled in the container.

In the step (d), the aqueous solution may be sterilized by filtering through a sterilizing filter.
In an embodiment, in the step (b), the organic solvent may be evaporated by a commonly employed method. Specifically, it may be evaporated by using a vacuum evaporator.
In an embodiment, the aqueous solution used in the step (c) may be distilled water, physiological saline, or an aqueous solution of a freeze-drying adjuvant.
In the step (f), one or more selected from a group consisting of mannitol, sorbitol, lactic acid, trehalose and sucrose may be used as a freeze-drying adjuvant. Specifically, mannitol may be used.
In accordance with the preparation method according to this embodiment, a micelle comprising an amphiphilic diblock copolymer, a micelle comprising an amphiphilic diblock copolymer and a polylactic acid alkali metal salt, a micelle comprising an amphiphilic diblock copolymer and a polylactic acid salt having a carboxyl terminus to which a divalent or trivalent metal ion is bound, or a mixture thereof can be prepared.

<Preparation of anti-cancer agent composition comprising mixture of taxane-containing polymer micelle or nanoparticle and cyclosporin-containing polymer micelle or nanoparticle>

This composition is a mixed type wherein a taxane-containing polymer micelle or nanoparticle and a cyclosporin-containing polymer micelle or nanoparticle are uniformly mixed.
The mixed micelle composition is prepared by mixing the polymer micelle compositions containing the respective drugs.
A method for preparing an anti-cancer agent composition comprising a mixture of a taxane-containing polymer micelle composition and a cyclosporin-containing polymer micelle composition may comprise:

(a) solubilizing taxane and cyclosporin respectively together with an amphiphilic diblock copolymer and, optionally, a polylactic acid alkali metal salt having at least one terminal carboxyl group in an organic solvent selected from a group consisting of ethanol, methanol, propanol, acetone, acetonitrile, dichloromethane, chloroform, methyl ethyl ketone and ethyl acetate;
(b) evaporating the organic solvent from the respective resulting solutions to prepare a mixture wherein taxane and the polymer are uniformly mixed and a mixture wherein cyclosporin and the polymer are uniformly mixed; and
(c) adding an aqueous solution to the respective mixtures prepared in the step (b) to prepare a taxane-containing polymer micelle composition and a cyclosporin-containing polymer micelle composition, and then mixing the micelle compositions to prepare a mixed micelle composition.

In an embodiment, when a polylactic acid alkali metal salt having at least one terminal carboxyl group is used to prepare a nanoparticle to which a divalent or trivalent metal ion is bound, the preparation method may further comprise, after the step (c):

(c-1) adding a divalent or trivalent metal ion to the mixed polymer micelle so that it is bound to the terminal group of the polylactic acid salt.

In the step (c-1), an aqueous solution containing 0.001-2 M of a divalent or trivalent metal ion may be added to the aqueous solution of the mixed polymer micelle, and the resultant mixture may be slowly stirred at room temperature for 0.1-1 hour to bind the metal ion to the terminal group of the polylactic acid salt via ionic bonding.
In another embodiment, the preparation method may further comprise, after the step (c):

(d) sterilizing the resulting mixed micelle composition;
(e) filling the sterilized mixed micelle composition in a container; and
(f) freeze drying the mixed micelle composition filled in the container.

The steps (d) through (f) are post-treatment processes of the prepared composition.
In the step (b), the organic solvent may be evaporated by a commonly employed method. Specifically, it may be evaporated by using a vacuum evaporator.
In an embodiment, the aqueous solution used in the step (c) may be distilled water, physiological saline, or an aqueous solution of a freeze-drying adjuvant.
In the step (f), one or more selected from a group consisting of mannitol, sorbitol, lactic acid, trehalose and sucrose may be used as a freeze-drying adjuvant. Specifically, mannitol may be used.
In accordance with the preparation method according to this embodiment, a micelle comprising an amphiphilic

diblock copolymer, a micelle comprising an amphiphilic diblock copolymer and a polylactic acid alkali metal salt, a micelle comprising an amphiphilic diblock copolymer and a polylactic acid salt having a carboxyl terminus to which a divalent or trivalent metal ion is bound, or a mixture thereof can be prepared.

The anti-cancer agent composition for treatment of resistant cancer may further comprise a pharmaceutical adjuvant such as an antiseptic, a stabilizer, a hydration agent, an emulsion accelerator, a salt and/or buffer for osmotic pressure control, etc. or other therapeutically useful substances, and may be prepared into formulations for oral or parenteral administration according to commonly employed methods.

Formulations for parenteral administration may be administered rectally, topically, transdermally, intravenously, intramuscularly, intraabdominally, subcutaneously, or the like. A typical example is an isotonic aqueous solution or suspension for injection. In an embodiment of the present disclosure, the anti-cancer agent composition for treatment of resistant cancer may be prepared into a freeze-dried formulation and may be administered by intravenous injection after reconstructing in distilled water for injection, 5% glucose solution, physiological saline, or the like.

An acceptable dose of cyclosporin for human is 190-230 $mg/m^3$/day, and it may be administered slowly via intravenous dropwise injection. For paclitaxel as an example of the taxane, Taxol® may be administered slowly via intravenous dropwise injection (175 $mg/m^3$ over 3 hours), and Abraxane® may be administered slowly via intravenous dropwise injection (300 $mg/m^3$ over 3 hours).

The anti-cancer agent composition according to the present disclosure, which is a complex or mixed polymer composition comprising taxane effective for resistant cancer caused by overexpression of P-glycoprotein and cyclosporin as a P-glycoprotein inhibitor, exhibits a significantly improved effect on resistant cancer in a resistant cancer-transplanted animal.

## EXAMPLES

The examples and experiments will now be described. The following examples and experiments are for illustrative purposes only and not intended to limit the scope of this disclosure.

An amphiphilic diblock copolymer and a polylactic acid alkali metal salt having at least one terminal carboxyl group were prepared according to the method disclosed in Korean Patent Application NO. 2005-7020313.

[Example 1] Preparation of complex anti-cancer agent micelle composition comprising amphiphilic diblock copolymer wherein paclitaxel and cyclosporin are encapsulated together

A complex anti-cancer agent composition for treatment of resistant cancer comprising an amphiphilic monomethoxypolyethylene glycol-polylactide (mPEG-PLA) diblock copolymer wherein paclitaxel and cyclosporin are encapsulated together was prepared.

After solubilizing the substances described in Table 1 in 10 mL of an organic solvent mixture comprising dichloromethane and methanol (1:1), the organic solvent was evaporated using a vacuum evaporator to prepare a composition wherein paclitaxel, cyclosporin and the polymer are uniformly mixed. To thus prepared composition, an aqueous solution was added so that the final concentration of paclitaxel was 3 mg/mL to prepare a polymer micelle composition wherein paclitaxel and cyclosporin are encapsulated together. The resulting composition was filtered through a sterilizing filter, transferred to a glass vial, and then free dried after adding 100 mg of mannitol.

Table 1

| | mPEG-PLA (mg) | Paclitaxel (PTX, mg) | Cyclosporin (CyA, mg) | PTX/CyA |
|---|---|---|---|---|
| Composition 1 | 577.8 | 10 | 20 | 0.5 |
| Composition 2 | 400 | 10 | 10 | 1.0 |
| Number average molecular weight of mPEG-PLA = 2,000-1,500 daltons | | | | |

[Example 2] Preparation of complex anti-cancer agent micelle composition comprising amphiphilic diblock copolymer and polylactic acid alkali metal salt wherein paclitaxel and cyclosporin are encapsulated together

A complex anti-cancer agent micelle composition for treatment of resistant cancer comprising an amphiphilic mPEG-PLA diblock copolymer and a polylactic acid sodium salt (PLA-COONa) wherein paclitaxel and cyclosporin are encapsulated together was prepared.

After solubilizing the substances described in Table 2 in an organic solvent mixture comprising dichloromethane, and methanol.(1:1), the organic solvent was evaporated using a vacuum evaporator to prepare a composition wherein

paclitaxel, cyclosporin and the polymer are uniformly mixed. To thus prepared composition, an aqueous solution was added so that the final concentration of paclitaxel was 3 mg/mL to prepare a complex polymer micelle composition wherein paclitaxel and cyclosporin are encapsulated together. The resulting composition was filtered through a sterilizing filter, transferred to a glass vial, and then free dried after adding 100 mg of mannitol.

Table 2

| | mPEG-PLA (mg) | PLA-COONa (mg) | PTX (mg) | CyA (mg) | PTX/CyA |
|---|---|---|---|---|---|
| Composition 3 | 400 | 177.78 | 10 | 20 | 0.5 |
| Composition 4 | 276.9 | 123.11 | 10 | 10 | 1.0 |
| Composition 5 | 400 | 177.78 | 20 | 10 | 2.0 |
| Number average molecular weight of mPEG-PLA = 2,000-1,500 daltons<br>Number average molecular weight of PLA-COONa = 1,300 daltons | | | | | |

[Example 3] Preparation of complex polymer micelle composition wherein docetaxel and cyclosporin are encapsulated together

A micelle composition comprising an amphiphilic diblock copolymer and a polylactic acid alkali metal salt wherein docetaxel and cyclosporin are encapsulated together was prepared in the same manner as in Examples 1 and 2, except for using docetaxel instead of paclitaxel. Details are shown in Table 3.

Table 3

| | mPEG-PLA (mg) | Docetaxel (DTX, mg) | CyA (mg) | DTX/CyA |
|---|---|---|---|---|
| Composition 6 | 577.8 | 10 | 20 | 0.5 |
| Composition 7 | 400 | 10 | 10 | 1.0 |

[Example 4] Preparation of complex polymer nanoparticle composition wherein docetaxel and cyclosporin are encapsulated together

After solubilizing the substances described in Table 4 in an organic solvent mixture comprising dichloromethane and methanol (1:1), the organic solvent was evaporated using a vacuum evaporator to prepare a composition wherein docetaxel, cyclosporin and the polymer are uniformly mixed. To thus prepared composition, an aqueous solution was added so that the final concentration of docetaxel was 3 mg/mL to prepare a complex polymer micelle composition wherein docetaxel and cyclosporin are encapsulated together. Then, after adding an aqueous solution of 0.2 M anhydrous calcium chloride, the mixture was stirred at room temperature for 20 minutes. Thus prepared nanoparticle composition was filtered through a sterilizing filter, transferred to a glass vial, and then free dried after adding 100 mg of mannitol.

Table 4

| | mPEG-PLA (mg) | PLA-COONa (mg) | DTX (mg) | CyA (mg) | $CaCl_2$ (mg) | DTX/CyA |
|---|---|---|---|---|---|---|
| Composition 8 | 400 | 177.78 | 10 | 20 | 11.54 | 0.5 |
| Composition 9 | 276.9 | 123.11 | 10 | 10 | 8.03 | 1.0 |
| Composition 10 | 400 | 177.78 | 20 | 10 | 11.54 | 2.0 |
| Number average molecular weight of mPEG-PLA = 2,000-1,500 daltons<br>Number average molecular weight of PLA-COONa = 1,300 daltons | | | | | | |

[Example 5] Preparation of mixed anti-cancer agent micelle composition comprising paclitaxel-containing polymer micelle and cyclosporin-containing polymer micelle

After preparing polymer micelle compositions wherein paclitaxel and cyclosporin are encapsulated respectively, the two micelle compositions were mixed to prepare an anti-cancer agent composition for treatment of resistant cancer

a) Preparation of polymer micelle composition comprising cyclosporin, amphiphilic diblock copolymer and polylactic acid alkali metal salt

A mixture comprising the followings was prepared.

| | |
|---|---|
| Cyclosporin | 50 mg |
| mPEG-PLA (2,000-1,500 daltons) | 750 mg |
| PLA-COONa (1,300 daltons) | 250 mg |

The mixture was solubilized in ethanol and then the organic solvent was evaporated using a vacuum evaporator. Then, an aqueous solution was added so that the final concentration of cyclosporin was 3 mg/mL to prepare a cyclosporin-containing polymer micelle composition.

b) Preparation of paclitaxel-containing amphiphilic diblock copolymer

| | |
|---|---|
| Paclitaxel | 50 mg |
| mPEG-PLA (2,000-1,500 daltons) | 2,500 mg |

The mixture was solubilized in ethanol and then the organic solvent was evaporated using a vacuum evaporator. Then, an aqueous solution was added so that the final concentration of paclitaxel was 3 mg/mL to prepare a paclitaxel-containing polymer micelle composition.

c) Preparation of mixed polymer micelle composition

A mixed polymer micelle composition was prepared by mixing the paclitaxel-containing polymer micelle composition and the cyclosporin-containing polymer micelle composition in an aqueous solution so that the weight proportion of paclitaxel/cyclosporin was 1.0.

Thus prepared mixed polymer micelle composition was filtered through a sterilizing filter, transferred to a glass vial, and then free dried after adding 100 mg of mannitol.

Particle size of the prepared composition was measured. The result is given in Table 5.

Table 5

| | | | |
|---|---|---|---|
| Composition 11 | Ex. 5, a) | Cyclosporin-containing polymer micelle composition | Particle size: 20-30 nm |
| Composition 12 | Ex. 5, b) | Paclitaxel-containing polymer micelle composition | Particle size: 17-24 nm |
| Composition 13 | Ex. 5, c) | Mixed micelle composition of paclitaxel-containing polymer micelle composition and cyclosporin-containing polymer micelle composition | Particle size: 17-30 nm |

[Example 6] Preparation of mixed nanoparticle composition comprising docetaxel-containing polymer nanoparticle composition and cyclosporin-containing polymer nanoparticle composition

After preparing polymer nanoparticle compositions wherein docetaxel and cyclosporin are encapsulated respectively, the two nanoparticle compositions were mixed to prepare mixed polymer nanoparticle composition.

a) Preparation of cyclosporin-containing polymer nanoparticle composition

A nanoparticle to which a divalent metal ion is bound was prepared as follows. A mixture comprising the followings was prepared.

| | |
|---|---|
| Cyclosporin | 20 mg |
| mPEG-PLA (2,000-1,500 daltons) | 300 mg |

(continued)

| | |
|---|---|
| D,L-PLA-COONa (1,300 daltons) | 60 mg |

The mixture was solubilized in ethanol in the same manner as in Example 2 and then the organic solvent was evaporated using a vacuum evaporator. An aqueous solution was added to the resultant so that the final concentration of cyclosporin was 3 mg/mL. Then, 3.9 mg of $CaCl_2$ was added to prepare a cyclosporin-containing polymer nanoparticle composition.

b) Preparation of docetaxel-containing nanoparticle composition

| | |
|---|---|
| Docetaxel | 20 mg |
| mPEG-PLA (2,000-1,500 daltons) | 500 mg |
| D,L-PLA-COONa (1,300 daltons) | 167 mg |

Docetaxel was completely dissolved in ethanol. Then, after adding the polymer, the resulting mixture was solubilized until complete dissolution. Then, after adding an aqueous solution of 10.89 mg of $CaCl_2$, the mixture was completely mixed using an electromagnetic mixer.

c) Preparation of mixed nanoparticle composition comprising docetaxel-containing polymernanoparticle composition and cyclosporin-containing polymer nanoparticle composition

A mixed polymer nanoparticle composition was prepared by mixing the docetaxel-containing polymer nanoparticle composition and the cyclosporin-containing polymer nanoparticle composition in an aqueous solution so that the weight proportion of docetaxel/cyclosporin was 1.0.

Thus prepared mixed polymer nanoparticle composition was filtered through a sterilizing filter, transferred to a glass vial, and then free dried after adding 100 mg of mannitol. Particle size of the prepared composition was measured. The result is given in Table 6.

Table 6

| | | | |
|---|---|---|---|
| Composition 14 | Ex. 6, a) | Cyclosporin-containing polymer nanoparticle composition | Particle size: 20-30 nm |
| Composition 15 | Ex. 6, b) | Docetaxel-containing polymer nanoparticle composition | Particle size: 17-20 nm |
| Composition 16 | Ex. 6, c) | Mixed nanoparticle composition of docetaxel-containing polymer nanoparticle composition and cyclosporin-containing polymer nanoparticle composition | Particle size: 17-30 nm |

[Test Example 1] Particle size of complex micelle wherein paclitaxel and cyclosporin are encapsulated together

Composition 5 of Example 2 was reconstructed in physiological saline so that the final concentration of paclitaxel was 3 mg/mL and then diluted 20 times with the same solvent to prepare a sample for particle size measurement. The particle size of the diluted Composition 5 solution was measured using a particle size analyzer. The result is shown in FIG. 1.

As seen from FIG. 1, the polymer micelle had a particle size of 40-50 nm. The polymer micelle had a very uniform particle size distribution with a polydispersity index smaller than 0.200.

[Test Example 2] Stability of micelle wherein paclitaxel and cyclosporin are encapsulated together

The Composition 5 solution diluted in Test Example 1 was allowed to stand at room temperature for 24 hours in order to evaluate the stability of the solution. During the test period, the concentration of paclitaxel and cyclosporin

was measured by high-performance liquid chromatography (HPLC). The result is shown in FIG. 2 and the specific HPLC condition is as follows.

| Condition for paclitaxel concentration measurement | Condition for cyclosporin concentration measurement |
|---|---|
| Injection volume: 0.010 mL | Injection volume: 0.020 mL |
| Flow rate: 1.5 mL/min<br>Wavelength: 227 nm<br>Mobile phase: acetonitrile 55% + ultrapure water 45%<br>Column: 4.6 x 250 mm (C18, Vydac, USA)<br>Drug peak: 4.52 min | Flow rate: 2 mL/min<br>Wavelength: 215 nm<br>Mobile phase: acetonitrile 70% + ultrapure water 30%<br>Column: 4.6 x 150 mm (C18, Zorbax SB-CN, USA)<br>Column temperature: 60 °C<br>Drug peak: 1.376 min |

The release volume of paclitaxel and cyclosporin can be calculated by measuring their concentration in the polymer micelle. As seen from FIG. 2, the concentration of paclitaxel and cyclosporin was maintained above 98%, indicating that the two drugs were released within 2%.

[Test Example 3] Retention of micelle wherein paclitaxel and cyclosporin are encapsulated together in bloodstream and delivery of drug

Retention of Composition 5 wherein paclitaxel and cyclosporin are encapsulated together and delivery of paclitaxel to the brain were evaluated.

For animal test, 8-week-old ICR mice weighing 20-25 g were used, five per each group. Composition 5 of Example 2, Composition 12 of Example 5, or commercially available Taxol® for injection was injected to the tail vein, at a dose of 5 mg/kg based on paclitaxel. 10 and 30 minutes and 1, 5, 10, 24 and 48 hours after the injection, whole blood and brain tissue samples were taken from the mouse.

The whole blood sample was centrifuged and 0.1 mL of clear serum (supernatant) was put in a covered glass tube. The brain tissue sample was added to ultrapure water of about 4 times its weight, homogenized using a tissue homogenizer, and the same volume as that of the serum was put in a covered glass tube. To each sample, 0.1 mL of an acetonitrile solution containing an internal standard was added. After adding 10 mL of ethyl acetate to the solution and vigorously stirring the mixture for 30 seconds, centrifugation was performed at 2,500 ppm for 10 minutes. After transferring the whole ethyl acetate layer to a test tube, the organic solvent was completely evaporated at 40 °C under nitrogen flow. After adding 0.1 mL of a 40% (v/v) acetonitrile solution and vigorously stirring the mixture for 30 seconds, HPLC was carried out. The HPLC condition was as follows. The concentration of paclitaxel in the serum is shown in FIG. 3, and the delivery of the drug to the brain tissue is shown in FIG. 4.
Injection volume: 0.075 mL
Flow rate: 1.0 mL/min
Wavelength: 227 nm
Mobile phase: 24% acetonitrile aqueous solution for 5 minutes, increased to 58% for 16 minutes, increased to 70% for 2 minutes, decreased to 34% for 4 minutes, and maintained for 5 minutes
Column: 4.6 x 250 mm (C18, Vydac, USA).
[Test Example 4] Inhibition of P-glycoprotein

The $IC_{50}$ value of paclitaxel in DLD-1 colon cancer cells treated with 1.88 μg/mL cyclosporin, which is the concentration exhibiting the maximum P-glycoprotein inhibition effect without affecting cytotoxicity, decreased 15 times (from 160 ng/mL to 11 ng/mL) as compared to when paclitaxel was administered alone.

[Test Example 5] Retention of mixed amphiphilic diblock copolymer in bloodstream

Retention of Composition 12 and Composition 13 of Example 5 copolymer in bloodstream was compared.

For animal test, male Sprague-Dawley rats weighing 210-250 g were used. After intravenous injection at a dose of 5 mg/kg based on paclitaxel, 0.3 mL of whole blood sample was taken from the tail artery at 5, 15 and 30 minutes and 1, 3, 6, 8 and 20 hours after the injection. The whole blood sample was centrifuged and 0.1 mL of clear serum (supernatant) was put in a covered glass tube, and 0.1 mL of an acetonitrile solution containing an internal standard was added. After adding 10 mL of ethyl acetate to the solution and vigorously stirring the mixture for 30 seconds, centrifugation was performed at 2,500 ppm for 10 minutes. After transferring the whole ethyl acetate layer to a test tube, the organic solvent was completely evaporated at 40 °C under nitrogen flow. After adding 0.1 mL of a 40% (v/v) acetonitrile

solution and vigorously stirring the mixture for 30 seconds, HPLC was carried out. The HPLC condition was as follows. The concentration of paclitaxel in the serum is shown in FIG. 5.
Injection volume: 0.075 mL
Flow rate: 1.0 mL/min
Wavelength: 227 nm
Mobile phase: 24% acetonitrile aqueous solution for 5 minutes, increased to 58% for 16 minutes, increased to 70% for 2 minutes, decreased to 34% for 4 minutes, and maintained for 5 minutes
Column: 4.6 x 250 mm (C18, Vydac, USA)

As seen from FIG. 5, Composition 13 showed longer retention in bloodstream than Composition 12. This reveals that cyclosporin keeps the concentration of paclitaxel in blood higher.

[Test Example 6] Anti-cancer activity of mixed polymer micelle composition Anti-cancer activity of Compositions 11-13 of Example 5 was examined.

Cells that had been stored in liquid nitrogen were cultured in a test tube. The cells were harvested and washed with sterilized phosphate buffered saline (PBS). Then, the number of living cells was counted, and the cells were resuspended in sterilized PBS at a concentration of $7x10^7$ cells/mL.

0.1 mL of a cell suspension containing $7x10^6$ human resistant colon cancer cells (DLD-1) was subcutaneously injected to the right flank of a healthy athymic nude (nu/nu) mouse (20-25 g, 8 weeks old). After the cancer had grown to a certain size, xenotransplantation was performed 3 times to form a xenograft of 3-4 mm. The xenograft was subcutaneously injected to the right flank of a healthy athymic nude (nu/nu) mouse (20-25 g, 8 weeks old) using a 12-gauge troca needle. After the tumor volume reached 100-300 $m^3$, the drug was administered. The day on which the drug was administered was recorded as day 0. On day 0, the mice were divided into 5 groups. On days 0, 3 and 6, the cyclosporin-containing polymer micelle composition (Composition 11), the paclitaxel-containing polymer micelle composition (Composition 12) and the mixed polymer micelle composition (Composition 13) were each administered through the tail vein at a dose of 35 mg/kg paclitaxel and 35 mg/kg cyclosporin.

The tumor volume was measured with time. The tumor volume was calculated according to Equation 1.

Equation 1

$$\text{Tumor volume (TV)} = (W^2 \times L) / 2 \text{ (L: major axis, W: minor axis)}$$

For evaluation of therapeutic effect, the relative tumor volume was calculated according to Equation 2.

Equation 2

$$\text{Relative tumor volume (RTV)} = (V_t / V_o) \times 100\% \text{ (}V_t\text{: TV on day t, }V_o\text{: TV on day 0)}$$

In order to recognize the validity of the experiments, at least 4 mice were used per treatment and at least 4 tumors were used per group. At the starting point of the treatment, the tumor size was 4 mm in diameter and 30 $mm^3$ in volume. The animals that died within 2 weeks after the final drug administration were regarded as toxified death and excluded from the evaluation. The groups with more than one death out of 3 animals, attributable to toxified death, or the groups including animals that had an average body weight decrease of more than 15% and did not show signs of thorough recovery were considered to have no anti-cancer activity. The result is shown in FIG. 6.

As seen from FIG. 6, the group treated with the cyclosporin-containing polymer micelle composition (Composition 11) had no effect on the anti-cancer activity. On the contrary, the tumor volume increased when compared to the control group. In contrast, the groups treated with the paclitaxel-containing polymer micelle composition (Composition 12) or the mixed polymer micelle composition (Composition 13) showed improved inhibitory activity against cancer growth compared with the control group. Especially, the group treated with the mixed polymer micelle composition (Composition 13) showed better inhibitory activity against cancer growth than the group treated with the paclitaxel-containing polymer micelle composition (Composition 12).

## Claims

1. A polymer micelle composition for use in the treatment of resistant cancer by parenteral administration comprising:

   an amphiphilic diblock copolymer, preferably a diblock copolymer comprising a hydrophilic block and a hydrophobic block, wherein the hydrophilic block is one or more selected from a group consisting of polyethylene glycol and methoxypolyethylene glycol, and the hydrophobic block is one or more selected from a group consisting of polylactic acid, polylactide, polyglycolide, polymandelic acid, polycaprolactone, polydioxan-2-one, polyamino acid, polyorthoester, polyanhydride and a copolymer thereof;
   taxane, preferably paclitaxel, docetaxel, 7-epipaclitaxel, t-acetyl paclitaxel, 10-desacetyl paclitaxel, 10-desacetyl-7-epipaclitaxel, 7-xylosyl paclitaxel, 10-desacetyl-7-glutaryl paclitaxel, 7-N,N-dimethylglycyl paclitaxel, 7-L-alanyl paclitaxel or a mixture thereof, as active ingredients; and
   cyclosporine, a P-glycoprotein inhibitor, as active ingredients,
   wherein the composition is formulated for parenteral administration.

2. The polymer micelle composition for treatment of resistant cancer according to claim 1, wherein the composition further comprises a polylactic acid alkali metal salt having a terminal carboxyl group, preferably a polylactic acid having a carboxyl terminus to which a divalent or trivalent metal ion is bound.

3. The polymer micelle composition for treatment of resistant cancer according to any one of claims 1to 2, wherein the composition comprises taxane and cyclosporin together in one micelle.

4. The polymer micelle composition for treatment of resistant cancer according to any one of claims 1 to 3, wherein the composition comprises a mixture of a taxane-containing polymer micelle and a cyclosporin-containing polymer micelle.

5. The polymer micelle composition for treatment of resistant cancer according to any one of claims 1 to 4, wherein the composition comprises a mixture of:

   a composition comprising taxane and cyclosporin together in one micelle; and
   a composition comprising a mixture of a taxane-containing polymer micelle and a cyclosporin-containing polymer micelle.

6. The polymer micelle composition for treatment of resistant cancer according to any one of claims 1 to 5, wherein the hydrophilic block has a number average molecular weight of 500-20,000 daltons, the hydrophobic block has a number average molecular weight of 500-10,000 daltons, and the content of the hydrophilic block is 40-70 wt% based on the total weight of the diblock copolymer.

7. The polymer micelle composition for treatment of resistant cancer according to any one of claims 1 to 6, wherein the weight ratio of taxane to cyclosporin is from 0.1 to 2.0.

8. The polymer micelle composition for treatment of resistant cancer according to any one of claims 1 to 7, wherein the combined content of taxane and cyclosporin is 0.1-20 wt% based on the total weight of the composition.

9. The polymer micelle composition for treatment of resistant cancer according to any one of claims 1 to 8, wherein the composition comprises:

   0.01-10 wt% of taxane;
   0.01-10 wt% of cyclosporin; and
   80-99.8 wt% of an amphiphilic diblock copolymer,
   based on the total weight of the composition.

10. The polymer micelle composition for treatment of resistant cancer according to any one of claims 2 to 8, wherein the composition comprises:

   0.01-10 wt% of taxane;
   0.01-10 wt% of cyclosporin;
   40-90 wt% of an amphiphilic diblock copolymer; and

10-50 wt% of a polylactic acid alkali metal salt having a terminal carboxyl group,
based on the total weight of the composition.

**11.** A method for preparing a polymer micelle composition for use in the treatment of resistant cancer comprising taxane and cyclosporin as active ingredients, comprising:

solubilizing taxane, cyclosporin and an amphiphilic diblock copolymer in an organic solvent;
evaporating the organic solvent to prepare a mixture wherein taxane, cyclosporinand the polymer are uniformly mixed; and
adding an aqueous solution to the mixture to prepare a polymer micelle wherein taxane and cyclosporin are encapsulated.

**12.** The method for preparing a polymer micelle composition for treatment of resistant cancer according to claim 11, which further comprises solubilizing a polylactic acid alkali metal salt having at least one terminal carboxyl group in the organic solvent in said solubilizing taxane, cyclosporin and an amphiphilic diblock copolymer in an organic solvent.

**13.** The method for preparing a polymer micelle composition for treatment of resistant cancer according to claim 11 or 12, which further comprises, after said preparing the polymer micelle, adding a divalent or trivalent metal ion to the polymer micelle so that it is bound to a terminal group of the polylactic acid salt.

**14.** The method for preparing a polymer micelle composition for treatment of resistant cancer according to any one of claims 11 to 13, wherein the weight ratio of taxane to cyclosporin is from 0.1 to 2.0.

**15.** The method for preparing a polymer micelle composition for treatment of resistant cancer according to any one of claims 11 to 14, which further comprises, after said preparing the polymer micelle, freeze drying the resulting micelle composition.

## Patentansprüche

**1.** Polymermizellenzusammensetzung zur Verwendung bei der Behandlung eines resistenten Krebses durch parenterale Verabreichung, umfassend:

ein amphiphiles Diblockcopolymer, vorzugsweise ein Diblockcopolymer, umfassend einen hydrophilen Block und einen hydrophoben Block, wobei der hydrophile Block eine oder mehrere Komponenten umfasst, die aus einer Gruppe bestehend aus Polyethylenglycol und Methoxypolyethylenglycol ausgewählt sind, und der hydrophobe Block eine oder mehrere Komponenten umfasst, die aus einer Gruppe bestehend aus Polymilchsäure, Polylactid, Polyglycolid, Polymandelsäure, Polycaprolacton, Polydioxan-2-on, Polyaminosäure, Polyorthoester, Polyanhydrid und ein Copolymer davon ausgewählt ist;
Taxan, vorzugsweise Paclitaxel, Docetaxel, 7-Epipaclitaxel, t-Acetylpaclitaxel, 10-Desacetylpaclitaxel, 10-Desacetyl-7-epipaclitaxel, 7-Xylosylpaclitaxel, 10-Desacetyl-7-glutarylpaclitaxel, 7-N,N-Dimethylglycylpaclitaxel, 7-L-Alanylpaclitaxel oder ein Gemisch daraus als Wirkstoffe; und
Cyclosporin, ein P-Glycoproteinhemmer als Wirkstoffe,
wobei die Zusammensetzung für die parenterale Verabreichung formuliert wird.

**2.** Polymermizellenzusammensetzung zur Behandlung eines resistenten Krebses nach Anspruch 1, wobei die Zusammensetzung weiter ein Polymilchsäurealkalimetallsalz mit einer terminalen Carboxylgruppe, vorzugsweise eine Polymilchsäure mit einem Carboxylterminus, an den ein zweiwertiges oder dreiwertiges Metallion gebunden ist, umfasst.

**3.** Polymermizellenzusammensetzung zur Behandlung eines resistenten Krebses nach einem der Ansprüche 1 bis 2, wobei die Zusammensetzung Taxan und Cyclosporin zusammen in einer Mizelle umfasst.

**4.** Polymermizellenzusammensetzung zur Behandlung eines resistenten Krebses nach einem der Ansprüche 1 bis 3, wobei die Zusammensetzung ein Gemisch aus einer Taxan enthaltenden Polymermizelle und einer Cyclosporin enthaltenden Polymermizelle umfasst.

**5.** Polymermizellenzusammensetzung zur Behandlung eines resistenten Krebses nach einem der Ansprüche 1 bis 4,

wobei die Zusammensetzung ein Gemisch aus:

einer Zusammensetzung, umfassend Taxan und Cyclosporin zusammen in einer Mizelle; und
einer Zusammensetzung, umfassend ein Gemisch aus einer Taxan enthaltenden Polymermizelle und einer Cyclosporin enthaltenden Polymermizelle, umfasst.

**6.** Polymermizellenzusammensetzung zur Behandlung eines resistenten Krebses nach einem der Ansprüche 1 bis 5, wobei der hydrophile Block ein Molekulargewicht-Zahlenmittel von 500 - 20.000 Dalton hat, der hydrophobe Block ein Molekulargewicht-Zahlenmittel von 500- 10.000 Dalton hat und der Gehalt des hydrophilen Blocks 40-70 Gew.-%, bezogen auf das Gesamtgewicht des Diblockcopolymers, beträgt.

**7.** Polymermizellenzusammensetzung zur Behandlung eines resistenten Krebses nach einem der Ansprüche 1 bis 6, wobei das Gewichtsverhältnis von Taxan zu Cyclosporin 0,1 bis 2,0 beträgt.

**8.** Polymermizellenzusammensetzung zur Behandlung eines resistenten Krebses nach einem der Ansprüche 1 bis 7, wobei der kombinierte Gehalt an Taxan und Cyclosporin 0,1 - 20 Gew.-%, bezogen auf das Gesamtgewicht der Zusammensetzung, beträgt.

**9.** Polymermizellenzusammensetzung zur Behandlung eines resistenten Krebses nach einem der Ansprüche 1 bis 8, wobei die Zusammensetzung umfasst:

0,01 - 10 Gew.-% Taxan;
0,01 - 10 Gew.-% Cyclosporin; und
80 - 99,8 Gew.-% eines amphiphilen Diblockcopolymers,
bezogen auf das Gesamtgewicht der Zusammensetzung.

**10.** Polymermizellenzusammensetzung zur Behandlung eines resistenten Krebses nach einem der Ansprüche 2 bis 8, wobei die Zusammensetzung umfasst:

0,01 - 10 Gew.-% Taxan;
0,01 - 10 Gew.-% Cyclosporin;
40 - 90 Gew.-% eines amphiphilen Diblockcopolymers; und
10 - 50 Gew.-% eines Polymilchsäurealkalimetallsalzes mit einer terminalen Carboxylgruppe,
bezogen auf das Gesamtgewicht der Zusammensetzung.

**11.** Verfahren zum Herstellen einer Polymermizellenzusammensetzung zur Verwendung bei der Behandlung eines resistenten Krebses, umfassend Taxan und Cyclosporin als Wirkstoffe, umfassend:

Lösen von Taxan, Cyclosporin und einem amphiphilen Diblockcopolymer in einem organischen Lösungsmittel,
Verdampfen des organischen Lösungsmittels zum Herstellen eines Gemischs, wobei Taxan, Cyclosporin und das Polymer gleichförmig gemischt werden: und
Zugeben einer wässrigen Lösung zu dem Gemisch, um eine Polymermizelle herzustellen, wobei Taxan und Cyclosporin verkapselt sind.

**12.** Verfahren zum Herstellen einer Polymermizellenzusammensetzung zur Behandlung eines resistenten Krebses nach Anspruch 11, das weiter umfasst: Lösen eines Polymilchsäurealkalimetallsalzes mit mindestens einer terminalen Carboxylgruppe im organischen Lösungsmittel, in dem Taxan, Cyclosporin und einem amphiphilen Diblockcopolymer in einem organischem Lösungsmittel gelöst werden.

**13.** Verfahren zum Herstellen einer Polymermizellenzusammensetzung zur Behandlung eines resistenten Krebses nach Anspruch 11 oder 12, das nach dem Herstellen der Polymermizelle weiter das Zugeben eines zweiwertigen oder dreiwertigen Metallions zur Polymermizelle zum Binden an eine terminale Gruppe des Polymilchsäuresalzes umfasst.

**14.** Verfahren zum Herstellen einer Polymermizellenzusammensetzung zur Behandlung eines resistenten Krebses nach einem der Ansprüche 11 bis 13, wobei das Gewichtsverhältnis von Taxan zu Cyclosporin 0,1 bis 2,0 beträgt.

**15.** Verfahren zum Herstellen einer Polymermizellenzusammensetzung zur Behandlung eines resistenten Krebses nach

einem der Ansprüche 11 bis 14, das nach dem Herstellen der Polymermizelle weiter das Gefriertrocknen der sich ergebenden Mizellenzusammensetzung umfasst.

## Revendications

1. Composition de micelles polymères à utiliser pour le traitement par voie parentérale de cancers résistants, comprenant :

   un copolymère dibloc amphiphile, préférentiellement un copolymère dibloc comprenant un bloc hydrophile et un bloc hydrophobe, dans lequel le bloc hydrophile est au nombre de un ou plus, sélectionné parmi un groupe constitué de polyéthylène glycol et de méthoxypolyéthylène glycol, et le bloc hydrophobe est au nombre de un ou plus, sélectionné parmi un groupe constitué d'acide polylactique, de polylactide, de polyglycolide, d'acide polymandélique, de polycaprolactone, de polydioxan-2-one, de polyaminoacide, de polyorthoester, de polyanhydride et d'un copolymère qui en résulte ;
   du taxane, préférentiellement du paclitaxel, du docétaxel, du 7-épipaclitaxel, du t-acétyl paclitaxel, du 10-désacétyl paclitaxel, du 10-désacétyl-7-épipaclitaxel, du 7-xylosyl paclitaxel, du 10-désacétyl-7-glutaryl paclitaxel, du 7-N,N-diméthylglycyl paclitaxel, du 7-L-alanyl paclitaxel ou d'un mélange qui en résulte, en tant que principes actifs ; et
   de la cyclosporine, un inhibiteur de la P-glycoprotéine, en tant que principes actifs,
   dans laquelle la composition est formulée pour une administration par voie parentérale.

2. Composition de micelles polymères pour le traitement de cancers résistants selon la revendication 1, dans laquelle la composition comprend, en outre, un sel de métal alcalin d'acide polylactique ayant un groupe carboxyle terminal, préférentiellement un acide polylactique ayant un carboxyle terminal auquel est lié un ion métallique divalent ou trivalent.

3. Composition de micelles polymères pour le traitement de cancers résistants selon l'une quelconque des revendications 1 à 2, dans laquelle la composition comprend du taxane et de la cyclosporine ensemble dans une même micelle.

4. Composition de micelles polymères pour le traitement de cancers résistants selon l'une quelconque des revendications 1 à 3, dans laquelle la composition comprend un mélange de micelles polymères contenant du taxane et de micelles polymères contenant de la cyclosporine.

5. Composition de micelles polymères pour le traitement de cancers résistants selon l'une quelconque des revendications 1 à 4, dans laquelle la composition comprend un mélange :

   d'une composition comprenant du taxane et de la cyclosporine ensemble dans une même micelle ;
   et
   d'une composition comprenant un mélange de micelles polymères contenant du taxane et de micelles polymères contenant de la cyclosporine.

6. Composition de micelles polymères pour le traitement de cancers résistants selon l'une quelconque des revendications 1 à 5, dans laquelle le bloc hydrophile a une masse moléculaire moyenne en nombre de 500 à 20 000 daltons, le bloc hydrophobe a une masse moléculaire moyenne en nombre de 500 à 10 000 daltons, et la teneur en bloc hydrophile est comprise entre 40 et 70% en masse par rapport à la masse totale du copolymère dibloc.

7. Composition de micelles polymères pour le traitement de cancers résistants selon l'une quelconque des revendications 1 à 6, dans laquelle la masse de taxane rapportée à la masse de cyclosporine est comprise entre 0,1 et 2,0.

8. Composition de micelles polymères pour le traitement de cancers résistants selon l'une quelconque des revendications 1 à 7, dans laquelle la teneur globale en taxane et cyclosporine est comprise entre 0,1 et 20% en masse par rapport à la masse totale de la composition.

9. Composition de micelles polymères pour le traitement de cancers résistants selon l'une quelconque des revendications 1 à 8, dans laquelle la composition comprend :

0,01 à 10% en masse de taxane ;
0,01 à 10% en masse de cyclosporine ; et
80 à 99,8% en masse d'un copolymère dibloc amphiphile,
par rapport à la masse totale de la composition.

**10.** Composition de micelles polymères pour le traitement de cancers résistants selon l'une quelconque des revendications 2 à 8, dans laquelle la composition comprend :

0,01 à 10% en masse de taxane ;
0,01 à 10% en masse de cyclosporine ;
40 à 90% en masse d'un copolymère dibloc amphiphile ; et
10 à 50% en masse d'un sel de métal alcalin d'acide polylactique ayant un groupe carboxyle terminal,
par rapport à la masse totale de la composition.

**11.** Méthode de préparation d'une composition de micelles polymères à utiliser pour le traitement de cancers résistants comprenant du taxane et de la cyclosporine en tant que principes actifs, comprenant des étapes suivantes :

solubilisation du taxane, de la cyclosporine et d'un copolymère dibloc amphiphile dans un solvant organique ;
évaporation du solvant organique pour préparer un mélange dans lequel le taxane, la cyclosporine et le polymère sont mélangés uniformément ; et
ajout d'une solution aqueuse au mélange pour préparer des micelles polymères dans lesquelles le taxane et la cyclosporine sont encapsulés.

**12.** Méthode de préparation d'une composition de micelles polymères pour le traitement de cancers résistants selon la revendication 11, qui comprend, en outre, dans ladite étape de solubilisation du taxane, de la cyclosporine et d'un copolymère dibloc amphiphile dans un solvant organique, une étape de solubilisation dans le solvant organique d'un sel de métal alcalin d'acide polylactique ayant au moins un groupe carboxyle terminal.

**13.** Méthode de préparation d'une composition de micelles polymères pour le traitement de cancers résistants selon la revendication 11 ou 12, qui comprend, en outre, après ladite étape de préparation de la micelle polymère, une étape d'ajout d'un ion métallique divalent ou trivalent à la micelle polymère de manière à la lier à un groupe terminal du sel d'acide polylactique.

**14.** Méthode de préparation d'une composition de micelles polymères pour le traitement de cancers résistants selon l'une quelconque des revendications 11 à 13, dans laquelle la masse de taxane rapportée à la masse de cyclosporine est comprise entre 0,1 et 2,0.

**15.** Méthode de préparation d'une composition de micelles polymères pour le traitement de cancers résistants selon l'une quelconque des revendications 11 à 14, qui comprend, en outre, après ladite étape de préparation des micelles polymères, une étape de lyophilisation de la composition de micelles résultante.

Fig 1

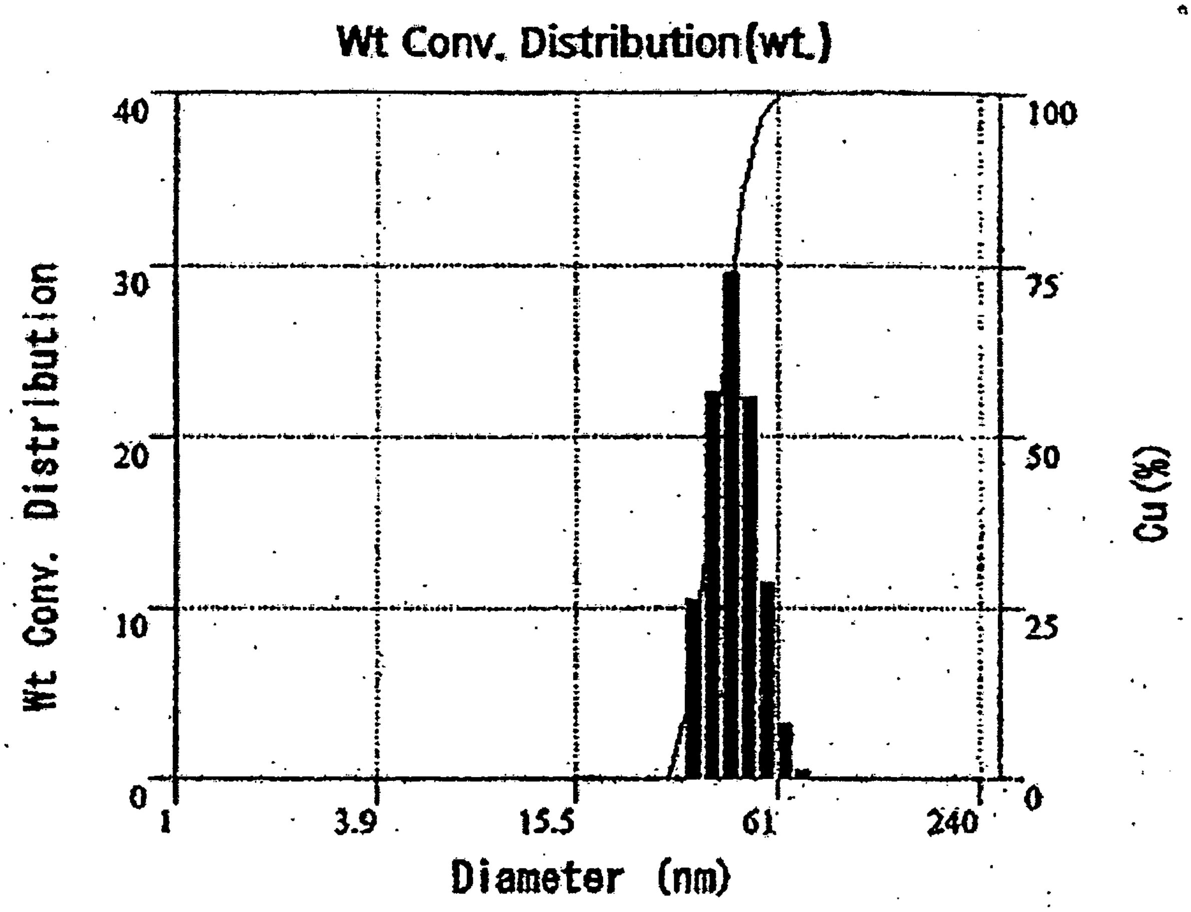
Wt Conv. Distribution(wt.)
Wt Conv. Distribution
Cu (%)
Diameter (nm)
40
30
20
10
0
100
75
50
25
0
1
3.9
15.5
61
240

Fig 2

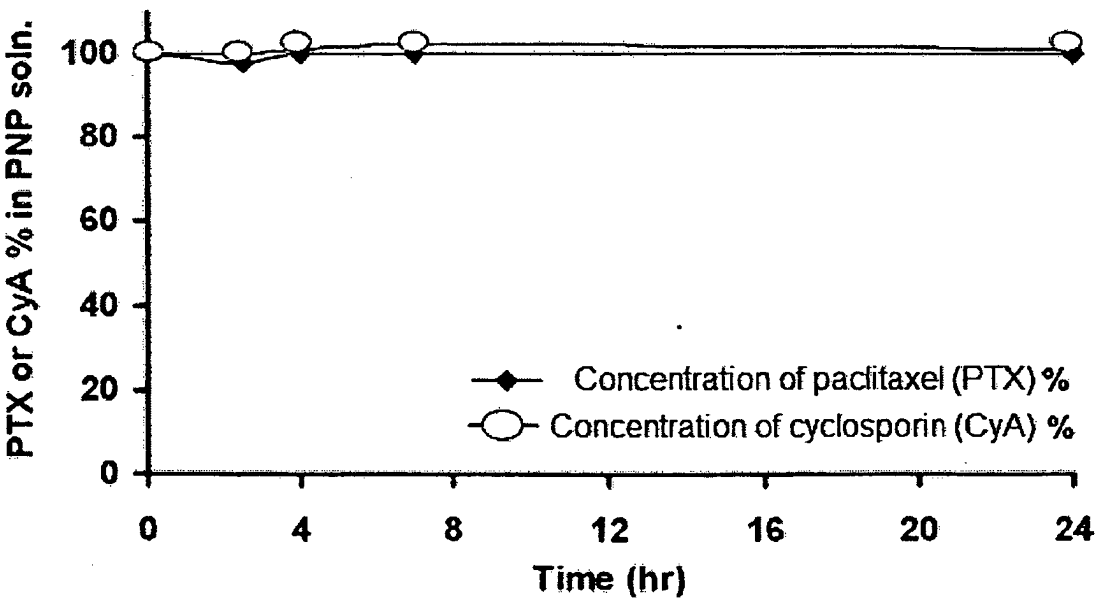
PTX or CyA % in PNP soln.
100
80
60
40
20
0
Concentration of paclitaxel (PTX) %
Concentration of cyclosporin (CyA) %
0
4
8
12
16
20
24
Time (hr)

Fig 3

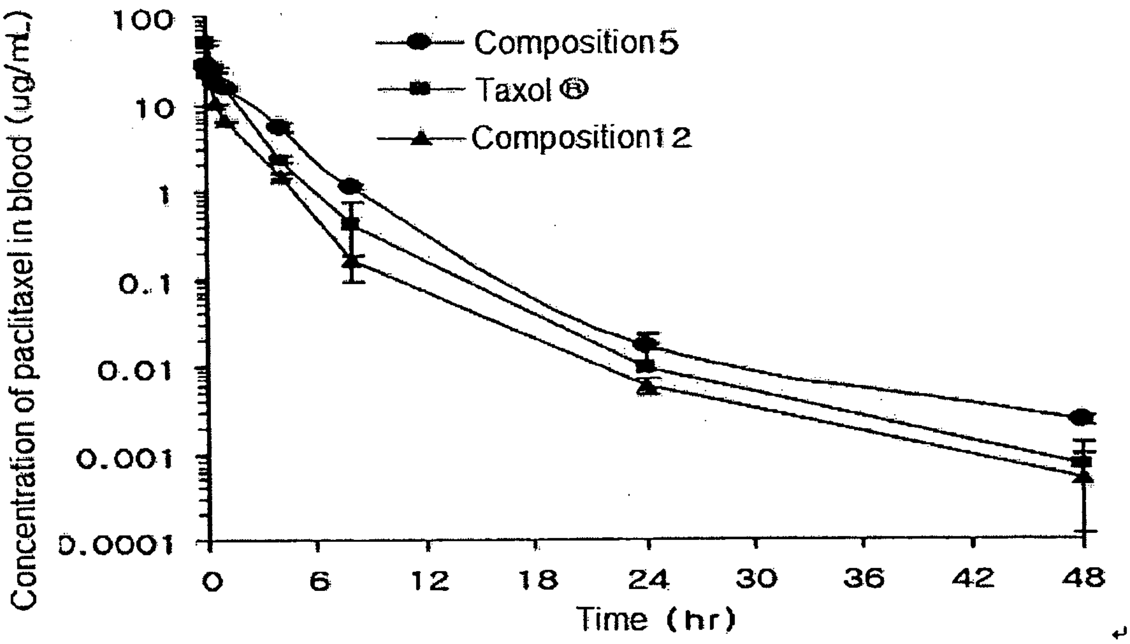
Concentration of paclitaxel in blood (ug/mL)
100
10
1
0.1
0.01
0.001
0.0001
0
6
12
18
24
30
36
42
48
Time (hr)
Composition 5
Taxol ®
Composition 12

Fig 4

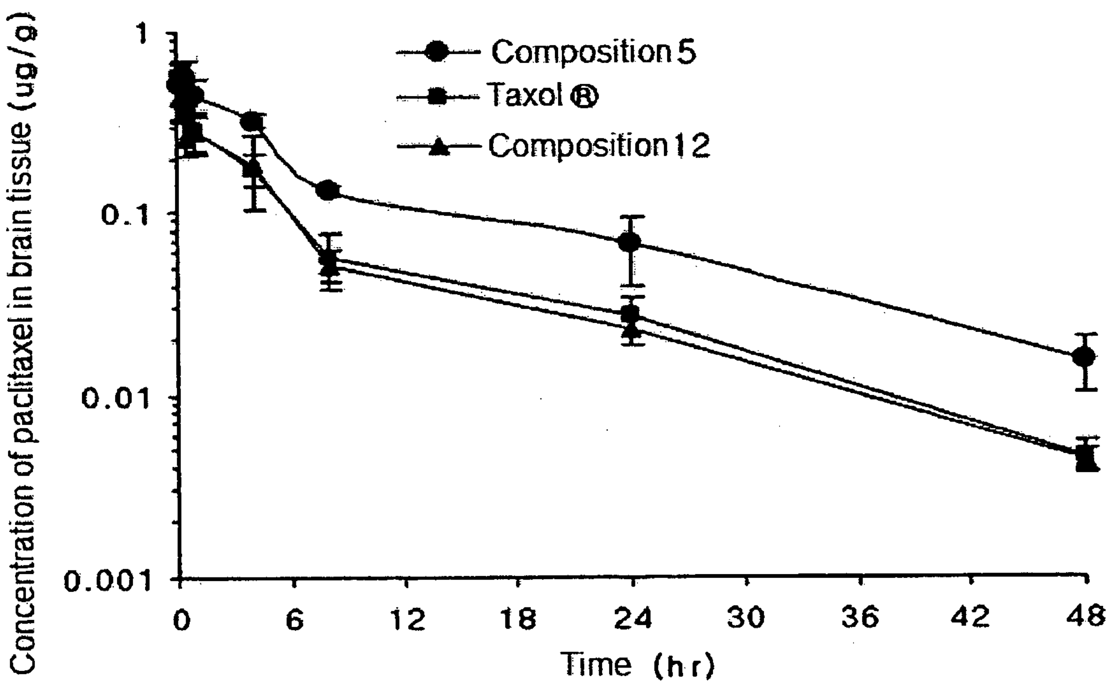
Concentration of paclitaxel in brain tissue (ug/g)
1
0.1
0.01
0.001
0
6
12
18
24
30
36
42
48
Time (hr)
Composition 5
Taxol ®
Composition 12

Fig 5

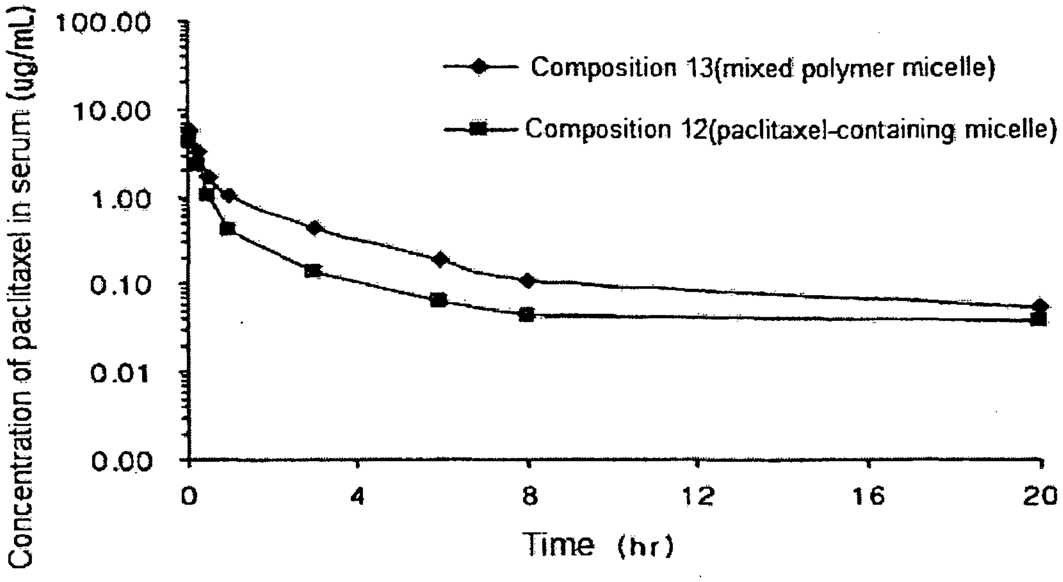
Concentration of paclitaxel in serum (ug/mL)
100.00
10.00
1.00
0.10
0.01
0.00
0
4
8
12
16
20
Time (hr)
Composition 13(mixed polymer micelle)
Composition 12(paclitaxel-containing micelle)

Fig 6

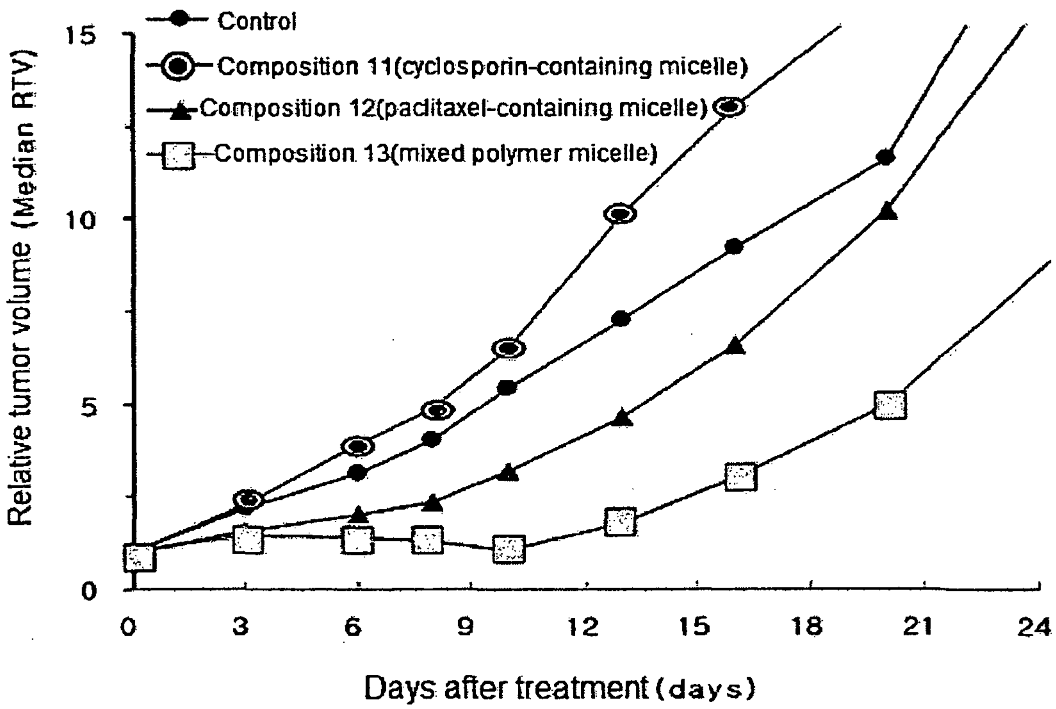
Relative tumor volume (Median RTV)
15
10
5
0
0
3
6
9
12
15
18
21
24
Days after treatment (days)
Control
Composition 11(cyclosporin-containing micelle)
Composition 12(paclitaxel-containing micelle)
Composition 13(mixed polymer micelle)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 20057020313